(19) Europäisches Patentamt
European Patent Office
Office européen des brevets

(11) Publication number: **0 121 614**
**B1**

(12) # EUROPEAN PATENT SPECIFICATION

(45) Date of publication of patent specification: **03.06.87**

(51) Int. Cl.$^4$: **C 07 D 209/58,** A 61 K 31/395

(21) Application number: **83301812.0**

(22) Date of filing: **30.03.83**

(54) **Perfluorotricyclic amine compounds.**

(43) Date of publication of application:
**17.10.84 Bulletin 84/42**

(45) Publication of the grant of the patent:
**03.06.87 Bulletin 87/23**

(84) Designated Contracting States:
**BE DE FR GB LU NL SE**

(56) References cited:
**FR-A-1 568 163**
**FR-A-2 218 903**
**US-A-3 993 581**
**US-A-4 105 798**

(73) Proprietor: **THE GREEN CROSS CORPORATION**
**15-1, Imabashi-1-chome Higashi-ku Osaka-shi**
**Osaka 541 (JP)**

(72) Inventor: **Yokoyama, Kazumasa**
**Sanhaitsu 2-201 7, Terauchi-2-chome**
**Toyonaka-shi (JP)**
Inventor: **Fukaya, Chikara**
**11-33-604, Kemacho-2-chome**
**Miyakojima-ku, Osaka-shi (JP)**
Inventor: **Tsuda, Yoshio**
**2-60, Isoshi-4-chome**
**Takarazuka-shi (JP)**
Inventor: **Ono, Taizo**
**c/o THE GREEN CROSS CORPORATION 1-47,**
**Chuo-1-chome**
**Joto-ku Osaka (JP)**
Inventor: **Arakawa, Yoshio**
**10-18-403, Esakacho-2-chome**
**Suita-shi (JP)**
Inventor: **Inoue, Yoshihisa**
**6, Nihonmatsucho Yoshida**
**Sakyo-ku Kyoto (JP)**
Inventor: **Naito, Youichiro**
**24-9, Kitanakafuri-1-chome**
**Hirakata-shi (JP)**
Inventor: **Suyama, Tadakazu**
**3-7, Matsuigaoka-4-chome**
**Tanabecho Tsuzuki-gun Kyoto (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 121 614 B1**

Courier Press, Leamington Spa, England.

0 121 614

(74) Representative: **Paget, Hugh Charles Edward et al**
**MEWBURN ELLIS & CO. 2/3 Cursitor Street**
**London EC4A 1BQ (GB)**

**Description**

This invention relates to a novel perfluorotricyclic amine compound useful as an oxygen carrier in "an artificial blood" or in an infusion fluid.

More particularly, it relates to perfluorotricyclic amine compounds represented by the general formula

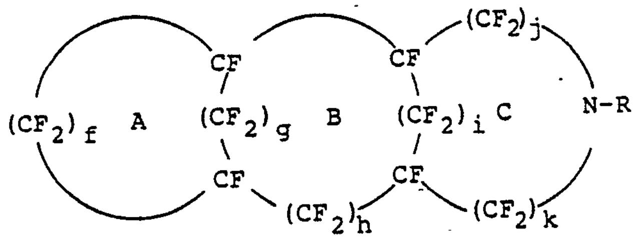

(I)

wherein R denotes a perfluoroalkyl group having from 1 to 4 carbon atoms, the ring A denotes a five- or six-membered ring, the ring B denotes a five-, six- or seven-membered ring, the ring C denotes a five- or six-membered ring, and f, g, h, i, j and k denote integers appropriately selected to construct the above-mentioned size of rings A, B and C. In that case, the compound may be substituted by one or more lower perfluoroalkyl group(s) in addition to the above-mentioned substituent R.

Regarding the general formula (I), as mentioned above, f, g, h, i, j and k are integers appropriately selected to construct a five- or six-membered ring A, a five-, six- or seven-membered ring B and a five- or six-membered ring C, respectively. Individually, each of the integers is appropriately selected, for example, from: 1, 2, 3 and 4 for f; 0, 1 and 2 for g; 0 and 1 for h; and 0, 1, 2 and 3 for each of i, j, and k.

In the general formula (I), the lower perfluoroalkyl group denoted by R may be either of straight chain or of branched chain. Examples thereof have 1 to 4, preferably 1 to 2, carbon atoms, such as perfluoromethyl group, perfluoroethyl group, perfluoro-p-propyl group, perfluoroisopropyl group, perfluoro-n-butyl group, perfluoroisobutyl group, perfluoro-sec-butyl group and perfluoro-tert-butyl group.

Rings A, B and C, especially rings A and C may be substituted at any position thereof by one or more than one, preferably one or two, lower perfluoroalkyl group(s) in addition to the above-mentioned substituent denoted by R. Examples of the lower perfluoroalkyl groups suitable as such substituents are similar to those which are described above referring to R. Perfluoromethyl group is especially preferred. In case where two or more of substituents are present, they may be different from each other.

The total number of carbon atoms present in the compound of formula (I) is from 8 to 12, preferably 10 or 11.

As an embodiment of the compound of formula (I), mention may be made of a compound represented by the general formula

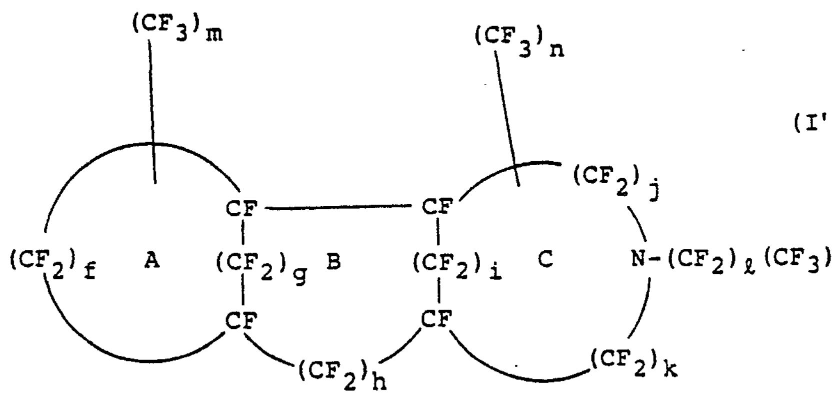

(I')

wherein rings A, B and C are as defined above; f + g + h + i + j + k + l + m + n = 5 or 6; f denotes 1, 2, 3 or 4; g denotes 0, 1 or 2; h, l, m and n denote each 0 or 1; and i, j and k denote each 0, 1, 2 or 3.

The compound of formula (I) can be prepared by fluorinating a perhydro-compound corresponding to the compound of formula (I). The methods of fluorination include, for example, a direct fluorination, a fluorination by use of cobalt trifluoride, and an electro chemical fluorination.

The preparation of the compound of formula (I) of this invention is preferably performed by the electrochemical fluorination method. This is performed, for example, by mixing anhydrous hydrogen fluoride and a perhydro-compound used as the starting compound in an electrolytic cell and subjecting the resulting solution to electrolysis. The voltage, the current density at the anode, and the temperature of electrolytic solution are normally 3—9 v, 0.2—3.0 A/$dm^2$ and 4—10°C, respectively.

The compound of formula (I) thus formed was drained from the bottom of the cell, being insoluble in anhydrous hydrogen fluoride.

The isolation and purification of the compound from the drained product are carried out, for example, by adding a mixture of equal volumes of an aqueous alkaline solution and an amine compound to the drained product, refluxing, then separating the lowermost layer containing the desired compound of formula (I) (the partially fluorinated compounds decomposed in this process), washing the former layer with an aqueous acetone solution containing a suitable amount of potassium iodide to remove perfluoroalkyl nitrogen fluorides, and by subsequent fractional distillation to obtain the fraction of the desired compound of formula (I).

Since the compound of the formula (I) of this invention can dissolve a large amount of oxygen, chemically and biologically inert, and can be excreted rapidly from the body, it can form, for example, an aqueous emulsion containing 5 to 50, preferably 10—40,% (W/V) of the compound of formula (I) to be used as an oxygen carrier in an artificial blood or in an infusion liquid for men and other mammals such as dogs, cats, cattle, mice, rats and guinea pigs.

The symbol "%(W/V)" referred to herein mean the amount of the material by weight (gram) based on 100 ml of the resulting emulsion.

In the preparation of the emulsion mentioned above, there used, as an emulsifier, a nonionic surfactant or phospholipids preferably in an added amount of 1 to 5% (W/V).

As the medium for the emulsion, a physiologically acceptable aqueous solution is employed. If necessary, there may be added thereto such materials as inorganic salts to provide the desired isotonicity, and such plasma expanders as HES or dextran to regulate the osmotic pressure of the emulsion.

The emulsion can be prepared by mixing the above-mentioned ingredients and homogenizing the mixture by means of, for example, a high-pressure jet type homogenizer until the particle diameters become 0.05 to 0.3 μm, preferably less than 0.2 μm.

The perhydro-compounds (starting compounds) corresponding to the compounds of formula (I) are substantially known already.

## Example

The equipment employed in the fluorination was a Monel reaction cell, through which coolant could be circulated for temperature control. Power was supplied to the cell pack by a 0—50 amp, 0—50 V·DC power supply. The cell pack consisted of 13 nickel plates (more than 99.6% purity) separated with Teflon (a Trade Mark) spacers, and arranged alternately so that the seven odd numbered plates were cathodes and the six even numbered plates were anodes.

The spacing between plates was 1.7—2.0 mm. The cell was equipped with a copper condenser, through which coolant was circulated by a refrigeration unit. The progress of an electrolysis was monitered by a voltage-current recorder. In the electrolytic cell, was placed 1.2 liter of hydrogen fluoride, and trace amounts of impurities present in the system (moisture or sulfuric acid) were removed by preliminary electrolysis. Then, 0.675 mol (102 g) of 4-methyl-4-azatricyclo[5,2,1,$0^{2,6}$]decane was introduced into the cell, and the electrolysis was continued, while introducing nitrogen gas from the bottom of the cell at a rate of 100 ml/min., under the conditions of anode current density of 0.4—2.0 A/$dm^2$, voltage of 5—7 V and solution temperature of 7—12°c, until the ampere-hours amounted to 730. Fresh anhydrous hydrogen fluoride (300ml) was added every 24 hours during the electrolysis. No attempt was made to collect volatile products formed by a bond breaking reaction. After completion of the electrolysis, fluorocarbons in the lower layer in the cell were drained through the bottom of the cell, weighed 260 g (57% yield).

To the fluorocarbons thus separated, were added equal volumes of 70% aqueous potassium hydroxide solution and diisobutylamine, and the resulting mixture was refluxed for about five days. The reaction mixture was then cooled in an ice bath. The perfluoro-compounds sedimented in the lowermost layer were separated in a separatory funnel, washed successively with water, concentrated sulfuric acid, saturated aqueous sodium hydrogen carbonate solution and 90% aqueous acetone solution containing 3% of potassium iodide, and finally several times with water to yield 162.0 g of perfluoro-compounds containing no protons. This was distilled on a fractional distillation apparatus equipped with a spinning band column to afford 39.2 g (8.6% yield) of the desired product boiling at 143—152°C This product was purified, then analyzed by infrared absorption spectrometry, $^{19}F$-nuclear magnetic resonance spectrometory and mass spectrometory, and was confirmed to be the objective compound, perfluoro-4-methyl-4-azatricyclo[5,2,1,$0^{2,6}$]decane.

A series of other perfluorotricyclic amine compounds were synthesized and purified in the same manner as that described above and each product was confirmed to be the objective compound upon analysis by infrared absorption spectrometory, $^{19}F$-nuclear magnetic resonance spectrometory and mass spectrometory.

The structural formula and the boiling point of each of the objective compounds is shown in the Table. The symbol "F-" in the structural formula indicates that the compound is perfluorinated. For example, the formula

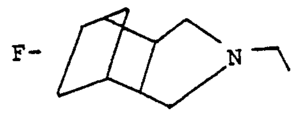

indicates in its excact meaning the formula

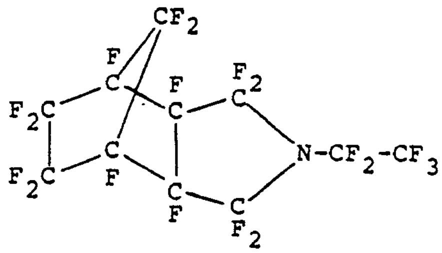

TABLE

| No. | Starting material Compound | Objective compound | Boiling point (°C) |
|---|---|---|---|
| 1 | 4-Methyl-4-azatricyclo[5,2,1,$0^{2,6}$]-decane | 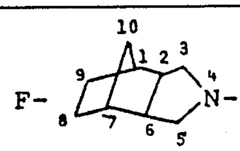 | 143—152 |
| 2 | 4-Ethyl-4-azatricyclo[5,2,1,$0^{2,6}$]-decane | 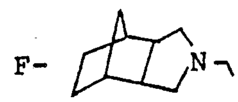 | 158—166 |
| 3 | 3,4-Dimethyl-4-azatricyclo-[5,2,1,$0^{2,6}$]decane | 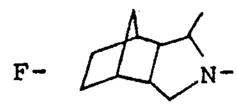 | 157—166 |
| 4 | 2,4-Dimethyl-4-azatricyclo-[5,2,1,$0^{2,6}$]decane | 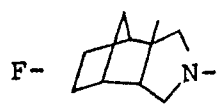 | 156—165 |
| 5 | 1,4-Dimethyl-4-azatricyclo-[5,2,1,$0^{2,6}$]decane | 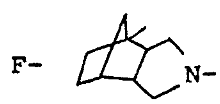 | 156—165 |
| 6 | 4,8-Dimethyl-4-azatricyclo-[5,2,1,$0^{2,6}$]decane | 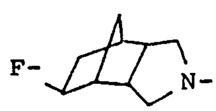 | 157—166 |
| 7 | 4,10-Dimethyl-4-azatricyclo-[5,2,1,$0^{2,6}$]decane | 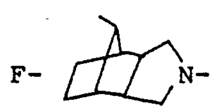 | 156—166 |
| 8 | 3-Methyl-3-azatricyclo-[5,2,1,$0^{2,6}$]decane | 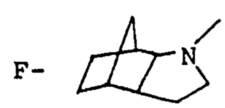 | 143—153 |
| 9 | 3-Ethyl-3-azatricyclo-[5,2,1,$0^{2,6}$]decane | 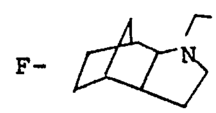 | 157—166 |

Table (Contined)

| No. | Starting material Compound | Objective compound | Boiling point (°C) |
|---|---|---|---|
| 10 | 1,3-Dimethyl-3-azatricyclo [5,2,1,$0^{2,6}$]decane | 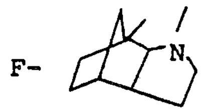 | 156—165 |
| 11 | 2,3-Dimethyl-3-azatricyclo-[5,2,1,$0^{2,6}$]decane | 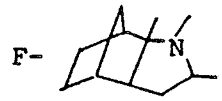 | 155—165 |
| 12 | 3,4-Dimethyl-3-azatricyclo-[5,2,1,$0^{2,6}$]decane | 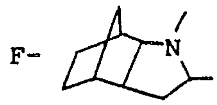 | 155—166 |
| 13 | 3,5-Dimethyl-3-azatricyclo-[5,2,1,$0^{2,6}$]decane | 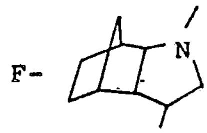 | 156—166 |
| 14 | 3,6-Dimethyl-3-azatricyclo-[5,2,1,$0^{2,6}$]decane | 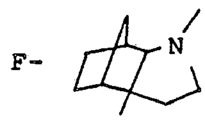 | 155—165 |
| 15 | 3,7-Dimethyl-3-azatricyclo-[5,2,1,$0^{2,6}$]decane | 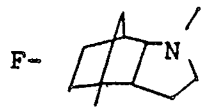 | 154—165 |
| 16 | 3,8-Dimethyl-3-azatricyclo-[5,2,1,$0^{2,6}$]decane | 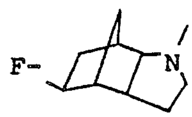 | 156—166 |
| 17 | 3,9-Dimethyl-3-azatricyclo-[5,2,1,$0^{2,6}$]decane | 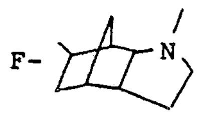 | 156—166 |
| 18 | 3,10-Dimethyl-3-azatricyclo-[5,2,1,$0^{2,6}$]decane | 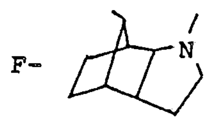 | 155—166 |
| 19 | 4-Methyl-4-azatricyclo-[6,2,1,$0^{2,7}$]undecane | 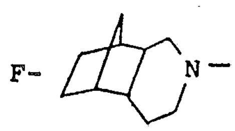 | 156—166 |
| 20 | 3-Methyl-3-azatricyclo-[6,2,1,$0^{2,7}$]undecane | 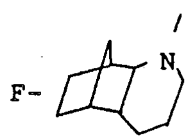 | 156—166 |
| 21 | 4-Methyl-4-azatricyclo-[5,2,1,$1^{2,6}$]undecane | 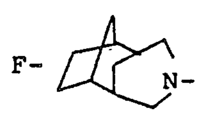 | 155—166 |
| 22 | 3-Methyl-3-azatricyclo-[5,2,1,$1^{2,6}$]undecane | 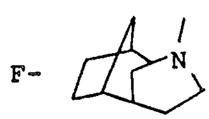 | 155—166 |

Table (Continued)

| No. | Starting material Compound | Objective compound | Boiling point (°C) |
|---|---|---|---|
| 23 | 4-Methyl-4-azatricyclo-[5,2,2,$0^{2,6}$]undecane | 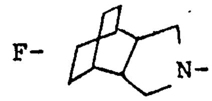 | 155—165 |
| 24 | 3-Methyl-3-azatricyclo-[5,2,2,$0^{2,6}$]undecane | 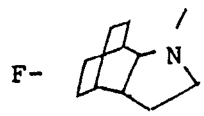 | 155—165 |
| 25 | 4-Methyl-4-azatricyclo-[5,3,1,$0^{2,6}$]undecane | 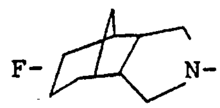 | 155—165 |
| 26 | 3-Methyl-3-azatricyclo-[5,3,1,$0^{2,6}$]undecane | 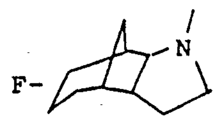 | 155—165 |
| 27 | 4-Methyl-4-azatricyclo-[6,2,1,$0^{2,6}$]undecane | 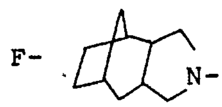 | 156—166 |
| 28 | 3-Methyl-3-azatricyclo-[6,2,1,$0^{2,6}$]undecane | 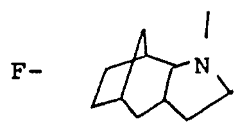 | 156—166 |
| 29 | 5-Methyl-5-azatricyclo-[6,2,1,$0^{2,6}$]undecane | 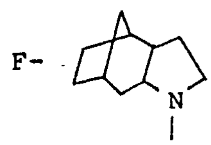 | 156—166 |
| 30 | 4-Methyl-4-azatricyclo-[6,3,0,$0^{2,6}$]undecane | 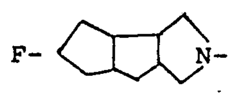 | 155—166 |
| 31 | 3-Methyl-3-azatricyclo-[6,3,0,$0^{2,6}$]undecane | 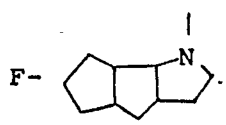 | 156—166 |
| 32 | 5-Methyl-5-azatricyclo-[6,3,0,$0^{2,6}$]undecane | 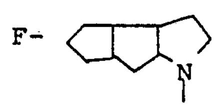 | 155—166 |
| 33 | 8-Methyl-8-azatricyclo-[5,3,1,$0^{2,6}$]undecane | 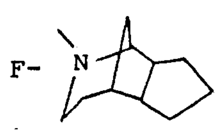 | 155—165 |
| 34 | 9-Methyl-9-azatricyclo-[5,3,1,$0^{2,6}$]undecane | 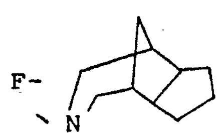 | 155—165 |

Table (Continued)

| No. | Starting material Compound | Objective compound | Boiling point (°C) |
|---|---|---|---|
| 35 | 3-Methyl-3-azatricyclo-[4,2,1,1$^{2,5}$]decane | 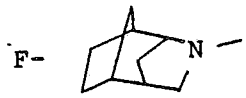 | 143—153 |
| 36 | 3-Ethyl-3-azatricyclo-[4,2,1,1$^{2,5}$]decane | 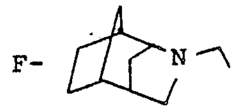 | 157—167 |
| 37 | 1,3-Dimethyl-3-azatricyclo-[4,2,1,1$^{2,5}$]decane | 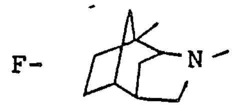 | 156—166 |
| 38 | 2,3-Dimethyl-3-azatricyclo-[4,2,1,1$^{2,5}$]decane | 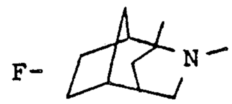 | 156—166 |
| 39 | 3,4-Dimethyl-3-azatricyclo-[4,2,1,1$^{2,5}$]decane | 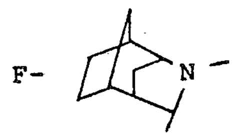 | 156—166 |
| 40 | 3,5-Dimethyl-3-azatricyclo-[4,2,1,1$^{2,5}$]decane | 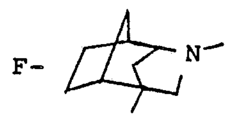 | 155—165 |
| 41 | 3,6-Dimethyl-3-azatricyclo-[4,2,1,1$^{2,5}$]decane | 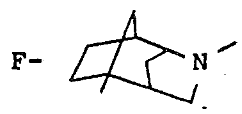 | 155—165 |
| 42 | 3,7-Dimethyl-3-azatricyclo-[4,2,1,1$^{2,5}$]decane | 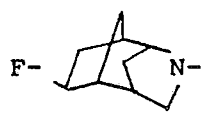 | 155—166 |
| 43 | 3,8-Dimethyl-3-azatricyclo-[4,2,1,1$^{2,5}$]decane | 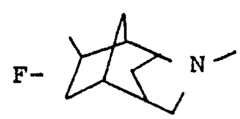 | 156—166 |
| 44 | 3,9-Dimethyl-3-azatricyclo-[4,2,1,1$^{2,5}$]decane | 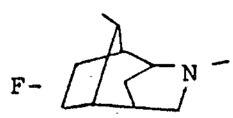 | 156—166 |
| 45 | 3,10-Dimethyl-3-azatricyclo-[4,2,1,1$^{2,5}$]decane | 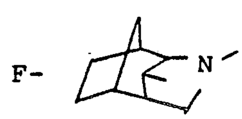 | 156—166 |
| 46 | 3-Methyl-3-azatricyclo-[5,2,1,1$^{2,5}$]undecane | 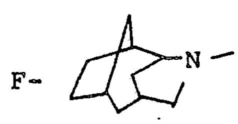 | 156—167 |
| 47 | 4-Methyl-4-azatricyclo-[5,2,1,1$^{2,6}$]undecane | 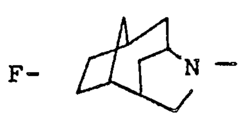 | 156—167 |

Table (Continued)

| No. | Starting material. Compound | Objective compound | Boiling point (°C) |
|---|---|---|---|
| 48 | 8-Methyl-8-azatricyclo-[5,2,1,$0^{2,6}$]decane | 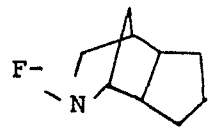 | 143—153 |
| 49 | 8-Ethyl-8-azatricyclo-[5,2,1,$1^{2,6}$]decane | 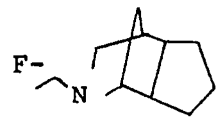 | 156—167 |
| 50 | 1,8-Dimethyl-8-azatricyclo-[5,2,1,$0^{2,6}$]decane | 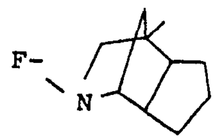 | 155—167 |
| 51 | 2,8-Dimethyl-8-azatricyclo-[5,2,1,$0^{2,6}$]decane | 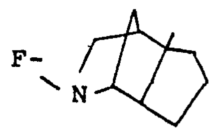 | 155—167 |
| 52 | 3,8-Dimethyl-8-azatricyclo-[5,2,1,$0^{2,6}$]decane | 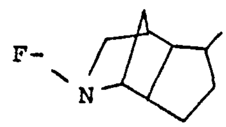 | 156—166 |
| 53 | 4,8-Dimethyl-8-azatricyclo-[4,2,1,$0^{2,6}$]decane | 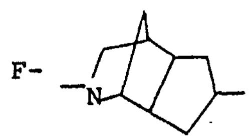 | 157—167 |
| 54 | 5,8-Dimethyl-8-azatricyclo-[5,2,1,$0^{2,6}$]decane | 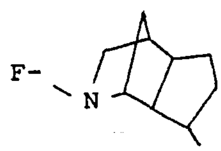 | 157—167 |
| 55 | 6,8-Dimethyl-8-azatricyclo-[5,2,1,$0^{2,6}$]decane | 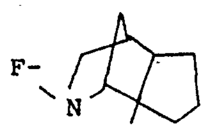 | 156—166 |
| 56 | 7,8-Dimethyl-8-azatricyclo-[5,2,1,$0^{2,6}$]decane | 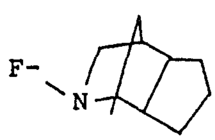 | 156—166 |
| 57 | 8,9-Dimethyl-8-azatricyclo-[5,2,1,$0^{2,6}$]decane | 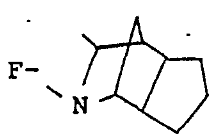 | 156—167 |
| 58 | 8,10-Dimethyl-8-azatricyclo-[5,2,1,$0^{2,6}$]decane | 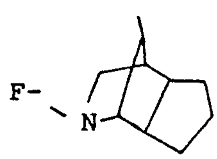 | 157—168 |

Table (Continued)

| No. | Starting material Compound | Objective compound | Boiling point (°C) |
|---|---|---|---|
| 59 | 9-Methyl-9-azatricyclo-[6,2,1,$0^{2,7}$]undecane | 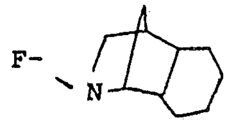 | 156—167 |
| 60 | 9-Methyl-9-azatricyclo-[6,2,1,$0^{2,6}$]undecane | 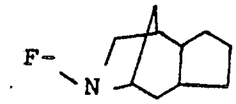 | 156—167 |
| 61 | 9-Methyl-9-azatricyclo-[6,2,1,$0^{3,7}$]undecane | 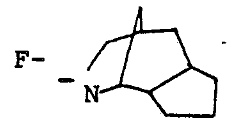 | 156—167 |
| 62 | 3-Methyl-3-azatricyclo-[4,3,1,$1^{2,5}$]undecane | 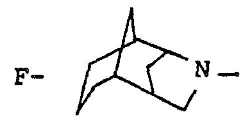 | 157—168 |
| 63 | 10-Methyl-10-azatricyclo-[5,2,2,$0^{2,6}$]undecane | 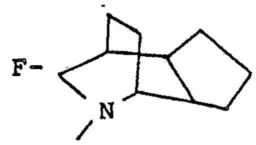 | 156—157 |
| 64 | 11-Methyl-11-azatricyclo-[5,3,1,$0^{2,6}$]undecane | 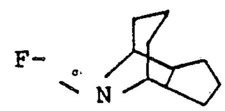 | 156—165 |
| 65 | 10-Methyl-10-azatricyclo-[5,2,1,$0^{2,6}$]decane | 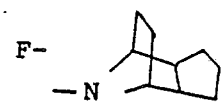 | 143—152 |
| 66 | 10-Ethyl-10-azatricyclo-[5,2,1,$0^{2,6}$]decane | 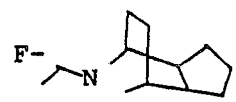 | 156—165 |
| 67 | 1,10-Dimethyl-10-azatricyclo-[5,2,1,$0^{2,6}$]decane | 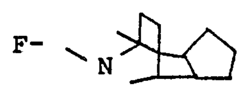 | 155—164 |
| 68 | 2,10-Dimethyl-10-azatricyclo-[5,2,1,$0^{2,6}$]decane | 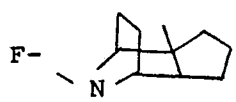 | 155—165 |
| 69 | 3,10-Dimethyl-10-azatricyclo-[5,2,1,$0^{2,6}$]decane | 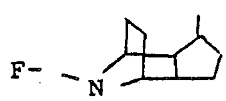 | 156—166 |
| 70 | 4,10-Dimethyl-10-azatricyclo-[5,2,1,$0^{2,6}$]decane | 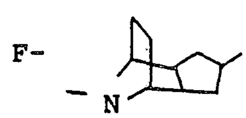 | 156—166 |
| 71 | 8,10-Dimethyl-10-azatricyclo-[5,2,1,$0^{2,6}$]decane | 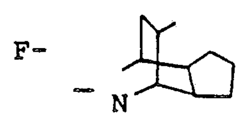 | 156—166 |

Table (Continued)

| No. | Starting material Compound | Objective compound | Boiling point (°C) |
|---|---|---|---|
| 72 | 11-Methyl-11-azatricyclo-$[6,2,1,0^{2,7}]$undecane | F- 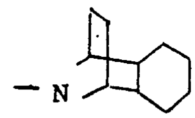 | 155—165 |
| 73 | 9-Methyl-9-azatricyclo-$[4,2,1,1^{2,5}]$decane | F- 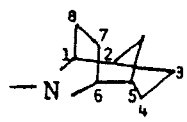 | 142—153 |
| 74 | 9-Ethyl-9-azatricyclo-$[4,2,1,1^{2,5}]$decane | F- 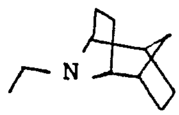 | 155—165 |
| 75 | 1,9-Dimethyl-9-azatricyclo-$[4,2,1,1^{2,5}]$decane | F- 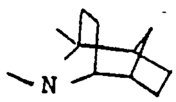 | 154—165 |
| 76 | 2,9-Dimethyl-9-azatricyclo-$[4,2,1,1^{2,5}]$decane | F- 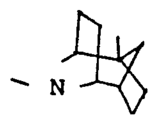 | 154—165 |
| 77 | 3,9-Dimethyl-9-azatricyclo-$[4,2,1,1^{2,5}]$decane | F- 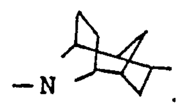 | 156—166 |
| 78 | 7,9-Dimethyl-9-azatricyclo-$[4,2,1,1^{2,5}]$decane | F- 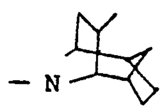 | 156—165 |
| 79 | 9,10-Dimethyl-9-azatricyclo-$[4,2,1,1^{2,5}]$decane | F- 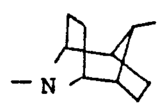 | 156—166 |
| 80 | 10-Methyl-10-azatricyclo-$[5,2,1,1^{2,6}]$undecane | F- 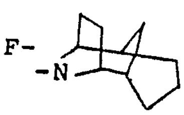 | 156—166 |
| 81 | 11-Methyl-11-azatricyclo-$[6,2,1,0^{2,6}]$undecane | F- 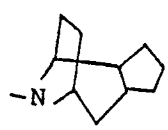 | 155—166 |
| 82 | 10-Methyl-10-azatricyclo-$[5,2,1,1^{2,5}]$undecane | F- 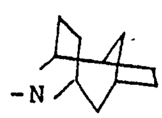 | 156—166 |

## Claims

1. A perfluorotricyclic amine compound having from 8 to 12 carbon atoms and being represented by the general formula

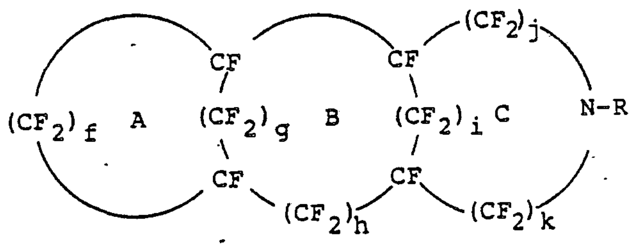

(I)

wherein R denotes a perfluoroalkyl group having from 1 to 4 carbon atoms; the ring A denotes a five- or six-membered ring, the ring B denotes a five-, six- or seven-membered ring, the ring C denotes a five- or six-membered ring, any one of the rings A, B and C optionally being substituted by one or more $C_{1-4}$ perfluoroalkyl group(s) in addition to the above-mentioned substituent R; and further f, g, h, i, j and k denote integers appropriately selected to construct the above-mentioned size of rings A, B and C.

2. A process for producing a perfluorotricyclic amine compound having from 8 to 12 carbon atoms and being represented by the formula,

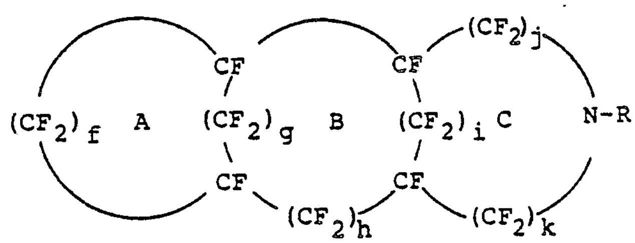

(I)

wherein R denotes a perfluoroalkyl group having from 1 to 4 carbon atoms, the ring A denotes a five- or six-membered ring, the ring B denotes a five-, six- or seven-membered ring, the ring C denotes a five- or six-membered ring, any one of the rings A, B and C optionally being substituted by one or more $C_{1-4}$ perfluoroalkyl group(s) in addition to the above-mentioned substituent R; and further f, g, h, i, j and k denote integers appropriately selected to construct the above-mentioned size of rings A, B and C, which comprises fluorinating a perhydro-compound of the formula,

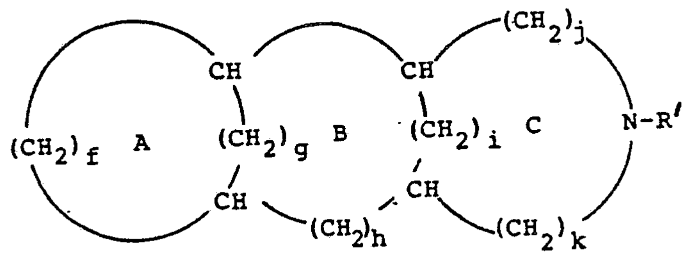

wherein R′ is an alkyl group having 1—4 carbon atoms, f, g, h, i, j and the rings A, B and C are as defined above and any of which is optionally substituted by a $C_{1-4}$ alkyl group.

3. A process according to claim 2, wherein fluorination of the perhydro-compound is effected by reaction thereof with fluorine.

4. A process according to claim 3, wherein the reaction with fluorine is effected by electrochemical fluorination of a mixture of the perhydro-compound with hydrogen fluoride.

5. A composition for use as a blood substitute or infusion fluid which composition is an aqueous emulsion of a compound of the formula (I) given and defined in claim 1.

6. A composition according to claim 5, which contains from 5 to 50% (w/v) of the compound of the formula (I).

7. A composition according to claim 6, which contains from 10 to 40% (w/v) of the compound of the formula (I).

8. A composition according to any one of claims 5 to 7, which contains, as emulsifier, a nonionic surfactant or phospholipid.

**Patentansprüche**

1. Eine perfluorotricyclische Aminverbindung mit 8 bis 12 Kohlenstoffatomen und der allgemeinen Formel

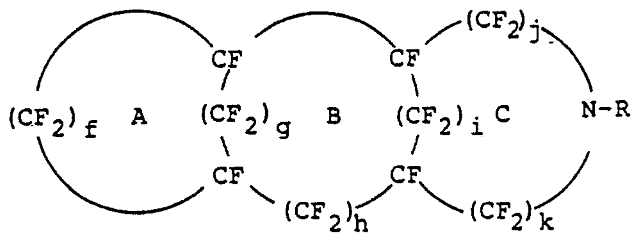

(I)

in der R einen Perfluoroalkylrest mit 1 bis 4 Kohlenstoffatomen bezeichnet, der Ring A einen 5- oder 6-gliedrigen Ring darstellt, der ring B einen 5-, 6- oder 7-gliedrigen Ring bedeutet, der Ring C einen 5- oder 6-gliedrigen Ring darstellt, wobei jeder der Ringe A, B und C gegebenenfalls durch mindestens einen $C_{1-4}$-Perfluoroalkylrest zusätzlich zu dem genannten Substituenten R substituiert sein kann, ferner f, g, h, i, j und k ganze Zahlen bedeuten, die in zum Aufbau der genannten Größe der Ringe A, B und C geeigneter Weise gewählt werden.

2. Verfahren zur Herstellung einer Perfluorotricyclischen Aminverbindung mit 8 bis 12 Kohlenstoffatomen der allgemeinen Formel

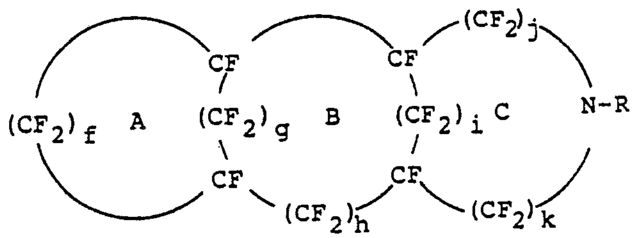

(I)

in der R einen Perfluoroalkylrest mit 1 bis 4 Kohlenstoffatomen bezeichnet, der Ring A einen 5- oder 6-gliedrigen Ring darstellt, der Ring B einen 5-, 6- oder 7-gliedrigen Ring bedeutet, der Ring C einen 5- oder 6-gliedrigen Ring darstellt, wobei jeder der Ringe A, B und C gegebenenfalls durch mindestens einen $C_{1-4}$-Perfluoroalkylrest zusätzlich zu dem genannten Substituenten R substituiert sein kann, ferner f, g, h, i, j und k ganze Zahlen bedeuten, die in zum Aufbau der genannten Größe der Ringe A, B und C geeigneter Weise gewählt werden, durch Fluorierung einer Perhydroverbindung der Formel

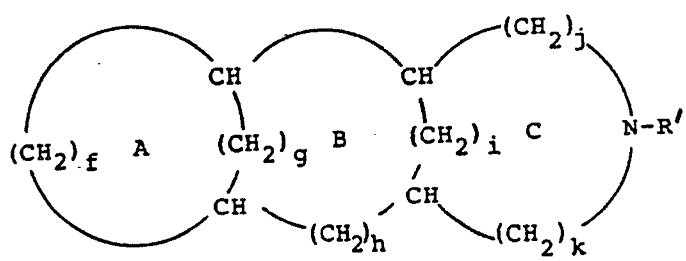

in der R′ einen Alkylrest mit 1 bis 4 Kohlenstoffatomen bedeutet, f, g, h, i, j und die Ringe A, B und C wie vorstehend definiert sind und jeder von ihnen gegebenenfalls durch einen $C_{1-4}$-Alkylrest substituiert sein kann.

3. Verfahren nach Anspruch 2, wobei die Fluorierung der Perhydroverbindung durch Umsetzung mit Fluor durchgeführt wird.

4. Verfahren nach Anspruch 3, wobei die Umsetzung mit Fluor durch elektrochemische Fluorierung eines Gemisches der Perhydroverbindung mit Fluorwasserstoff durchgeführt wird.

5. Zusammensetzung zur Verwendung als Blutersatzstoff oder Infusionsflüssigkeit, die eine wäßrige Emulsion einer Verbindung der Formel (I) gemäß Anspruch 1 darstellt.

6. Zusammensetzung nach Anspruch 5, die 5 bis 50% (Gew./Vol.) der Verbindung der Formel (I) enthält.

7. Zusammensetzung nach Anspruch 6, die 10 bis 40% (Gew./Vol.) der Verbindung der Formel (I) enthält.

8. Zusammensetzung nach einem der Ansprüche 5 bis 7, die als Emulgator ein nichtionisches grenzflächenaktives Mittel oder ein Phospholipid enthält.

**Revendications**

1. Amine perfluorotricyclique en $C_8$ à $C_{12}$ représentée par la formule générale:

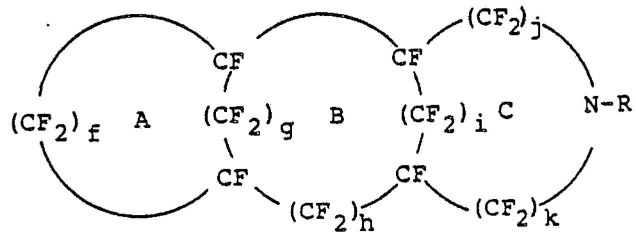

(I)

dans laquelle R désigne un groupe perfluoroalkyle en $C_1$ à $C_4$; le cycle A désigne un cycle à cinq ou six maillons, le cycle B désigne un cycle à cinq, six ou sept maillons, le cycle C désigne un cycle à cinq ou six maillons, l'un quelconque des cycles A, B et C étant substitué si on le désire par un ou plusieurs groupes perfluoroalkyle en $C_1$ à $C_4$ en plus du substituant R mentionné ci-dessus; et en outre f, g, h, i, j et k désignent des entiers adéquatement choisis pour construire la taille mentionnée ci-dessus des cycles A, B et C.

2. Procédé de préparation d'une amine perfluorotricyclique en $C_8$ à $C_{12}$, représenté par la formule

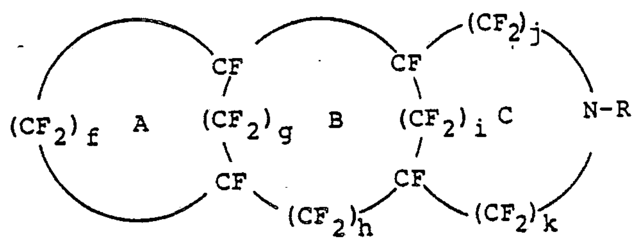

(I)

dans laquelle R désigne un groupe perfluoroalkyle en $C_1$ à $C_4$; le cycle A désigne un cycle à cinq ou six maillons, le cycle B désigne un cycle à cinq, six ou sept maillons, le cycle C désigne un cycle à cinq ou six maillons, l'un quelconque des cycles A, B et C étant substitué si on le désire par un ou plusieurs groupes perfluoroalkyle en $C_1$ à $C_6$ en plus du substituant R mentionné ci-dessus; et en outre f, g, h, i, j et k désignent des entiers adéquatement choisis pour construire la taille mentionnée ci-dessus des cycles A, B et C, qui consistent à fluorer un composé perhydro répondant à la formule:

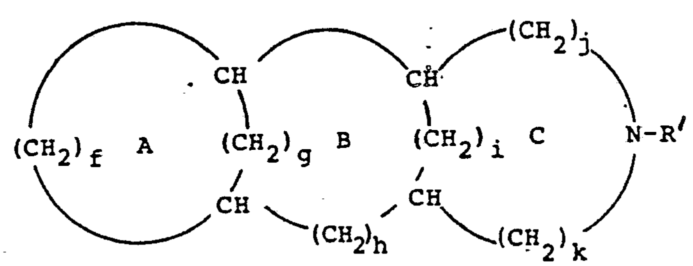

dans laquelle R' est un groupe alkyle en $C_1$ à $C_4$, f, g, h, i, j et les cycles A, B et C sont tels que définis ci-dessus, et l'un quelconque de ceux-ci est substitué si on le désire par un group alkyle en $C_1$ à $C_4$.

3. Procédé suivant la revendication 2, dans lequel la fluoration du composé perhydro est effectuée par réaction de celui-ci avec du fluor.

4. Procédé suivant la revendication 3, dans lequel la réaction avec le fluor est effectuée par fluoration électro-chimique d'un mélange du composé perhydro avec de l'acide fluorhydrique.

5. Composé destiné à être utilisé comme substitut du sang ou comme fluide pour perfusion, laquelle composition est une émulsion aqueuse d'un composé répondant à la formule (I) donnée et définie dans la revendication 1.

6. Composition suivant la revendication 5, qui contient de 5 à 50% (P/V) du composé répondant à la formule (I).

7. Composition suivant la revendication 6, qui contient de 10 à 40% (P/V) du composé répondant à la formule (I).

8. Composition suivant l'une quelconque des revendications 5 à 7, qui contient comme émulsionnant un agent tensio-actif non ionique ou un phospholipide.